# EUROPEAN PATENT APPLICATION

(11) **EP 2 072 043 A2**
(43) Date of publication of application: **24.06.2009**
(21) Application number: 08002649.5
(22) Date of filing: 13.02.2008
(51) Int. Cl.: A61K 9/28, A61K 31/606

(54) **Mesalazine tablet**

(30) Priority: 21.12.2007 US 963096
(71) Applicant: Disphar International B.V., 7255 PZ Hengelo (NL)
(72) Inventor: Ugwoke, Michael I., 1188 ER Amstelveen (NL); Corselli, Marta, 216 22 Limhamm (SE)
(74) Representative: Hirsch & Associés

(57) **Abstract**

Disclosed are mesalazine tablets and a method for their preparation. The mesalazine tablets comprise a tablet core, a first coating layer, and a second coating layer. The tablet core comprises mesalazine; the first coating layer comprises a cellulose derivative and/or povidone, and the second coating layer comprises methacrylic acid/methyl methacrylate copolymer and an anti-tack agent. The tablets show a high degree of uniformity and reproducibility and release mesalazine starting at pH 6.8 and are used in the treatment of colorectal diseases.

## Description

### BACKGROUND OF THE INVENTION

Mesalazine or mesalamine is an aminosalicylate that is prescribed for the treatment of Inflammatory Bowel Disease (IBD). IBD can manifest itself in a variety of forms, the most common of which are Crohn's disease and ulcerative colitis. Crohn's disease (CD) is a chronic transmural inflammation of the bowel which can affect the whole gastrointestinal tract, usually in a discontinuous pattern. The initial location of CD is most commonly in the lower ileum. From here the inflammation typically spreads towards proximal parts of the small intestine. However, the colon is also often involved. Ulcerative colitis is a chronic inflammatory bowel disease affecting only the colon and shows a continuous distribution in the gastrointestinal mucosa. In most patients mainly the distal part of the colon and the rectum are inflamed with often a proximal spread. In the most severe cases, the whole colon is affected ("pancolitis").

To date it is not possible to cure Crohn's disease or ulcerative colitis. Mesalazine plays an important role in the treatment of both diseases by inducing and maintaining remission in chronic inflammatory bowel diseases. Its main principle of action is a topical effect at the inflamed mucosa. Systemic absorption should be minimized, as this leads to unwanted systemic side-effects and inefficient redistribution of the mesalazine to the sites of inflammation. Therefore, oral mesalazine dosage forms should release the active substance selectively at the inflamed areas in the gastrointestinal tract. Because of the different disease patterns of Crohn's disease and ulcerative colitis, different formulations are required to adequately treat different patient subgroups.

From in vitro dissolution testing, however, it appears that currently marketed enteric coated tablets show a high degree of variation in release characteristics when tablets of several batches are compared (inter-batch variation), or when several tablets within a single batch are compared (intra-batch variation).

The problem of tablets with high variation in release characteristics is that these tablets are inconsistent and hence unreliable in their delivery of the pharmaceutical active ingredient to the target region in the intestines, leading to ineffective treatment.

### BRIEF SUMMARY OF THE INVENTION

It is an object of the present invention to provide tablets that deliver the pharmaceutical active ingredient at the right location, in the right dose and in the right time frame. It has been found that tablets with a reduced inter- and intra- batch variation in release characteristics improves the effectiveness of the treatment. Reduced inter- and intra- batch variation will allow achieving a given amount of active ingredient at a given locus, and thus a more effective treatment. The invention provides a mesalazine delayed release tablet comprising a tablet core and two coating layers disposed on the core. The tablet core comprises mesalazine and at least one intergranular superdisintegrant. The first coating layer comprises a cellulose derivative and/or polyvinylpyrrolidone (or povidone); and the second coating layer comprises a methacrylic acid/methyl methacrylate copolymer and an anti-tack agent. The invention also provides a method of preparing mesalazine delayed release tablets. The invention further provides a method of treating a patient suffering from an inflammatory bowel disease. One of the features of the delayed release tablets of the invention is that the drug release characteristics of the tablets are uniform or substantially uniform; for example, only small or reduced variation among the tablets prepared in a single batch (intra-batch variation) and/or small or reduced variation among tablets prepared in different batches (inter-batch variation) is seen. The tablets have only one layer of methyl methacrylic acid/methyl methacrylate copolymer.

The invention thus provides, for example,
(a) a mesalazine delayed release tablet comprising a tablet core, a first coating layer being in contact with and covering the tablet core, and a second coating layer being in contact with and covering the first coating layer, wherein
   the tablet core comprises mesalazine, a binder, and at least one intergranular superdisintegrant;
   the first coating layer which is free or substantially free of methacrylic acid/methyl methacrylate copolymer and which comprises a cellulose derivative and/or povidone; and
   the second coating layer comprises a methacrylic acid/methyl methacrylate copolymer and an anti-tack agent, wherein the amount of anti-tack agent is present in an amount about 40% to about 60% by weight of the methacrylic acid/methyl methacrylate copolymer;
(b) a batch of delayed release tablets comprising a plurality of delayed release mesalazine tablets, wherein the batch shows an intra-batch variation dt₅₀ less than about 200 minutes, wherein dt₅₀ is the difference in the time required to release 50% of mesalazine from the quickest tablet to the slowest tablet when 6 tablets of said batch are tested for dissolution in a phosphate buffer at pH 7.0 and 37°C according to the USP paddle dissolution test method;
(c) a set comprising multiple batches of delayed release tablets of mesalazine, each of said multiple batches of delayed release tablets comprising a plurality of mesalazine tablets, the set showing an inter-batch variation DT₅₀ less than about 150 minutes, wherein DT₅₀ is the difference in the average time to release 50% of the mesalazine (at₅₀) between the slowest batch and the quickest batch when 3 batches are tested for dissolution in a phosphate buffer at pH 7.0 and 37°C according to the USP paddle dissolution test method, wherein the average time of release of tablets from batch "j": at^{j}₅₀ = Σ(tⁱ₅₀)/6, and i=1 to 6, wherein tⁱ₅₀ is the time to release 50% of the mesalazine from tablet "i" of batch "j" when 6 tablets of the batch "j" are tested for dissolution in a phosphate buffer at pH 7.0 and 37°C according to the USP paddle dissolution test method;
(d) a method for preparing a mesalazine delayed release tablet comprising:
   (i) granulating a granulate composition comprising mesalazine and a binder to obtain mesalazine granulates;
   (ii) tabletting a core composition comprising the mesalazine granulates obtained in (i) and an intergranular superdisintegrant to obtain a tablet core;
   (iii) coating the tablet core obtained in (ii) with a first coating layer composition comprising a cellulose derivative and/or povidone to obtain a product; and
   (iv) coating the product obtained in (iii) with a second coating layer by employing a coating composition comprising a methacrylic acid/methyl methacrylate copolymer and an anti-tack agent and wherein the amount of anti-tack agent is about 40% to about 60% by weight of the methacrylic acid/methyl methacrylate copolymer; and optionally repeating steps (i) to (iv);
(e) in one embodiment, the tablet, batch of tablets or set of multiple batches of the invention are for the treatment of a patient with an inflammatory bowel disease (IBD), wherein the treatment comprises administering to the patient an effective amount of a mesalazine delayed release tablet/batch/set according to the invention; and
(f) in one embodiment, a mesalazine delayed release tablet comprising a tablet core comprising mesalazine and an intergranular superdisintegrant, a first coating layer and a second coating layer disposed on the tablet core, the first coating layer comprising hydroxypropyl methylcellulose and the second coating layer comprising poly(methacrylic acid, methyl methacrylate) 1:2 and about 40 to about 60% of talc based on the weight of poly(methacrylic acid, methyl methacrylate) 1:2.
Embodiments are the subject of dependant claims.

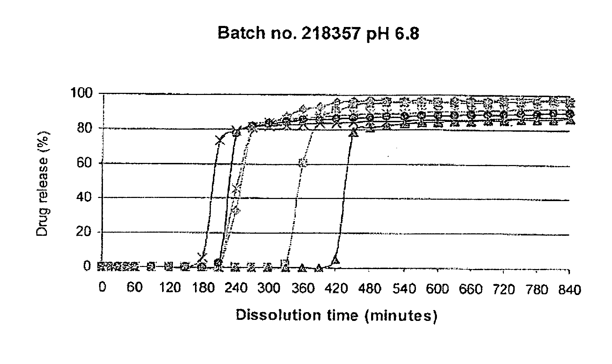

### BRIEF DESCRIPTION OF THE DRAWING

The Figure depicts the percentage of drug release in a dissolution experiment carried out at pH 6.8 as a function of time in minutes for six mesalazine delayed release tablets in accordance with an embodiment of the invention.

### DETAILED DESCRIPTION OF EMBODIMENTS OF THE INVENTION

The invention provides delayed release mesalazine tablets. Embodiments of the tablets have attractive features such as reduced variation of the drug release profile among several tablets within a single batch and among tablets of different batches. The tablets are designed to release mesalazine starting at pH 6.8 and to continue to release at higher pH's, for example, at pH 7.0. Thus, mesalazine is released in the terminal ileum and the colon of the patient. The delayed release feature is obtained by providing an enteric coating layer that surrounds a coated tablet core.

The second coating layer is an enteric coating layer, i.e., a coating layer that is resistant to gastric acid degradation in the stomach and hence the tablet does not start to dissolve until after it has passed the stomach. The tablet of the present invention includes a tablet core, a first coating layer, and a second (or enteric) coating layer. Optionally, a third or polishing layer may be present.

In accordance with an embodiment, the invention provides a mesalazine delayed release tablet comprising a tablet core, a first coating layer that is in contact with and covering the tablet core, and a second coating layer that is in contact with and covering the first coating layer, wherein
the tablet core comprises mesalazine, a pharmaceutically acceptable salt or ester thereof, a binder, and at least one intergranular superdisintegrant;
the first coating layer is free or substantially free of methacrylic acid/methyl methacrylate copolymer and comprises a cellulose derivative and/or povidone; and
the second coating layer comprises a methacrylic acid/methyl methacrylate copolymer and an anti-tack agent.

The tablet core comprises mesalazine, a pharmaceutically acceptable salt or ester thereof, a binder, and at least one intergranular superdisintegrant. Any suitable amount of the drug can be present in the tablet core, e.g., in an amount about 100 mg or more, 200 mg or more, 300 mg or more; or, in an amount about 900 mg or less, 600 mg or less, 500 mg, or less. For example, mesalazine can be present in an amount about 100 mg to about 800 mg, preferably about 200 mg to about 600 mg, and more preferably about 350 mg to about 450 mg of the active agent per tablet core. Alternatively, mesalazine, salt or ester thereof can be present in an amount about 10% or more, about 20% or more, or about 30% or more, by weight, or about 95% or less, about 85% or less, or about 75% or less by weight of the tablet core. In embodiments, the mesalazine, salt or ester thereof is present in an amount about 30% to about 90%, preferably about 50% to about 80%, and more preferably about 75% to about 77% by weight of the tablet core.

In accordance with the invention, any suitable binder can be employed. The binder holds the components of the tablet core together. Examples of suitable binders include microcrystalline cellulose, polyvinylpyrrolidone (povidone), lactose, starch, hydroxypropyl cellulose, and mixtures thereof. Other examples of binders include hydroxypropyl methyl cellulose, low-substituted hydroxypropyl ether of cellulose, for example, a hydroxypropyl ether of cellulose having a degree of substitution from 5 to 16 mass % when determined on a dry basis (US Pharmacopoeia, 23rd Ed., pp 2253-2254), glucose, carboxymethylcellulose, dextrin, ethylcellulose, gelatin, pregelatinized starch, and mixtures thereof.

Any suitable amount of binder can be employed to prepare the tablet core. For example, the binder is present in the core in an amount about 20 mg, 30 mg, or 40 mg or more; or in amount of 150 mg or less, 120 mg or less, or 100 mg or less, and in embodiments, in an amount about 50 mg to about 140 mg, preferably about 60 mg to about 100 mg, and more preferably about 70 mg to about 75 mg per tablet core. Alternatively, the binder can be present in an amount about 2% or more, about 4% or more, or about 8% or more, or in an amount about 30% or less, about 25% or less, or about 20% or less by weight of the tablet core. In certain embodiments, the binder is present in an amount about 5% to about 25%, preferably about 10% to about 20%, and more preferably about 15% to about 18% by weight of the tablet core.

In accordance with the invention, any suitable intergranular superdisintegrant can be present in the tablet core. A superdisintegrant is a substance in a tablet formulation that enables the tablet to break up in smaller fragments and is generally used at a low level in the solid dosage form, typically 1-10% by weight relative to the total weight of the dosage unit. Examples of suitable intergranular superdisintegrants include crospovidone, croscarmellose sodium which is crosslinked carboxymethyl cellulose sodium salt, and sodium starch glycollate and mixtures thereof, preferably crospovidone. Any suitable amount of the intergranular superdisintegrant can be employed. For example, the intergranular superdisintegrant is present in an amount about 10 mg or more, 20 mg or more, or 30 mg or more; or in amount of 35 mg or less, 25 mg or less, or 15 mg or less, and in embodiments, in an amount about 5 mg to about 40 mg, preferably about 10 mg to about 30 mg, and more preferably about 14 mg to about 27 mg per tablet core. Alternatively, the intergranular superdisintegrant can be present in an amount about 1% or more, about 4% or more, or about 6% or more, or in an amount about 8% or less, about 5% or less, or about 3% or less by weight of the tablet core. In certain embodiments, the intergranular superdisintegrant is present in an amount about 1% to about 10%, preferably about 2% to about 7%, and more preferably about 2% to about 5% by weight of the tablet core. In embodiments, the superdisintegrant can also be used as an intragranular superdisintegrant, i.e., wherein the superdisintegrant is used within the mesalazine granulate.

The tablet core can optionally include one or more excipients. These excipients include fillers, glidants, lubricants, and/or wetting agents. Suitable fillers include ethylcellulose and lactose. Suitable glidants include amorphous silica, powdered cellulose, and starch. Suitable wetting agents include sodium dodecyl sulfate (SDS).

Any suitable lubricant can be employed. A lubricant keeps the mixture from sticking to the equipment during the tabletting process. Examples of suitable lubricants include sodium stearyl fumarate (PRUV®), magnesium stearate, colloidal silicon dioxide, and talc. The lubricant can be present in any suitable amount, e.g., in an amount about 2 mg or more, about 4 mg or more, or about 6 mg or more; or in amount about 5 mg or less, about 3 mg or less, or about 1 mg or less, and in embodiments, in an amount about 2 mg to about 10 mg, preferably about 3 mg to about 7 mg, and more preferably about 4 mg to about 5 mg per tablet core. Alternatively, the lubricant can be present in an amount about 0.1 % or more, about 0.5% or more, or about 0.8% or more, or in an amount about 2% or less, about 1% or less, or about 0.5% or less, by weight of the tablet core. In certain embodiments, the lubricant is present in an amount about 0.5% to about 1%, and preferably about 0.8% to about 0.9% by weight of the tablet core.

According to the present invention, the tablet core is covered by a first coating layer and a second coating layer. It is believed that the first coating increases the adhesion of the second coating layer and/or removes any incompatibility between the tablet core and the second coating layer. The first coating layer is free or substantially free (e.g., less than 1%, 0.5%, or 0.1% by weight of the first coating layer) of a methacrylic acid/methyl methacrylate copolymer. The first coating layer comprises a cellulose derivative and/or polyvinylpyrrolidone (or povidone). Any suitable cellulose derivative can be employed, for example, a cellulose ether polymer, preferably a hydrophilic cellulose ether polymer such as hydroxypropyl methylcellulose. Optional additives can be present in the first coating layer, e.g., a polyol such as polyethylene glycol.

The first coating layer can be present in any suitable amount, for example, about 0.1 % or more, about 0.5% or more, about 0.8% or more, or in an amount about 2% or less, about 1% or less, or about 0.5% or less, by weight of the tablet. In embodiments, the first coating layer is present in an amount about 0.5% to about 1.5%, and preferably about 0.5% to about 0.9%, by weight of the tablet. Thus, for example, the first coating layer can be present in an amount about 2 mg or more, about 4 mg or more, or about 6 mg or more; or in amount about 5 mg or less, about 3 mg or less, or about 1 mg or less, and in embodiments, in an amount about 2 mg to about 10 mg, preferably about 3 mg to about 7 mg, and more preferably about 4 mg to about 5 mg per tablet.

The cellulose derivative can be present in the first coating layer in any suitable amount, for example, about 0.1% or more, about 0.4% or more, or about 0.7% or more, or in an amount about 2% or less, about 1% or less, or about 0.5% or less, by weight of the tablet. In certain embodiments, the cellulose derivative is present in an amount about 0.3% to about 1.5%, and preferably about 0.5% to about 0.8% by weight of the tablet. Thus, for example, the cellulose derivative can be present in an amount about 2 mg or more, about 4 mg or more, or about 6 mg or more; or in amount about 5 mg or less, about 3 mg or less, or about 1 mg or less, and in embodiments, in an amount about 2 mg to about 8 mg, preferably about 3 mg to about 6 mg, and more preferably about 3 mg to about 4 mg per tablet.

The optional additive, e.g., polyol, can be present in the first coating layer in any suitable amount, for example, 0.005% or more, about 0.1% or more, or about 0.15% or more, or in an amount about 0.2% or less, about 0.09% or less, or about 0.05% or less, by weight of the tablet. In certain embodiments, the optional additive is present in an amount about 0.001% to about 0.5%, and preferably about 0.05% to about 0.1% by weight of the tablet. Thus, for example, the optional additive can be present in an amount about 0.1 mg or more, about 0.3 mg or more, or about 0.5 mg or more; or in amount about 1 mg or less, about 0.5 mg or less, or about 0.2 mg or less, and in embodiments, in an amount about 0.2 mg to about 0.8 mg, preferably about 0.3 mg to about 0.7 mg, and more preferably about 0.4 mg to about 0.6 mg per tablet.

The first coating layer can be of any suitable loading, e.g., a loading of about 0.8 mg/cm² or more, and in embodiments, the first coating layer can have a loading of about 1 mg/cm² to about 2 mg/cm², preferably about 1.2 mg/cm² to about 1.8 mg/cm², and more preferably about 1.3 mg/cm² to about 1.4 mg/cm².

In accordance with the invention, the second coating layer comprises, consists essentially of (e.g., comprises more than 90% by weight especially more than 95% by weight) or consists of, a methacrylic acid/methyl methacrylate copolymer and an anti-tack agent. The second coating layer is an enteric coating layer. Optionally, the second coating layer can include additional components, e.g., plasticizers and coloring agents such as pigments, lakes, and dyes. Suitable plasticizers include triethyl citrate, diethyl phthalate, triethyl acetyl citrate, triacetin, tributyl citrate, dibutyl phthalate, polyethylene glycol, glycerol, and mixtures thereof. Suitable coloring agents include aluminum lakes, titanium dioxides, iron oxides or natural colors such as riboflavin or carotenoids.

The second coating layer can be present in any suitable amount, for example, about 1% or more, about 5% or more, or about 8% or more, or in an amount about 9% or less, about 6% or less, or about 5% or less, by weight of the tablet. In any of the embodiments of the invention, the second coating layer is present in an amount about 3% to about 10%, preferably about 4% to about 8%, and more preferably about 5% to about 7% by weight of the tablet. Thus, for example, the second coating layer can be present in an amount about 10 mg or more, about 30 mg or more, or about 50 mg or more; or in amount about 45 mg or less, about 35 mg or less, or about 25 mg or less; and in embodiments, in an amount about 25 mg to about 45 mg, preferably about 30 mg to about 40 mg, and more preferably about 32 mg to about 38 mg per tablet.

In accordance with an embodiment of the invention, the first coating layer is present in an amount of about 0.5% to about 1.5% by weight of the tablet and the second coating layer is present in an amount of about 3% to about 10% by weight of the tablet.

Any suitable methacrylic acid/methyl methacrylate copolymer can be used in the second coating layer. For example, the methacrylic acid/methyl methacrylate copolymer is methacrylic acid/methyl methacrylate copolymer (1:2) (e.g., Ph. Eur.), available under the trade name EUDRAGIT® S (Rohm Pharma GmbH, Germany).

The methacrylic acid/methyl methacrylate copolymer can be present in any suitable amount, for example, about 1% or more, about 3% or more, or about 5% or more, or in an amount about 6% or less, about 4% or less, or about 3% or less, by weight of the tablet. In certain embodiments, the methacrylic acid/methyl methacrylate copolymer is present in an amount about 1% to about 8%, preferably about 2% to about 5%, and more preferably about 3% to about 4% by weight of the tablet. Thus, for example, the methacrylic acid/methyl methacrylate copolymer can be present in an amount about 10 mg or more, about 30 mg or more, or about 50 mg or more; or in amount about 40 mg or less, about 30 mg or less, or about 20 mg or less, and in embodiments, in an amount about 12 mg to about 30 mg, preferably about 15 mg to about 25 mg, and more preferably about 16 mg to about 21 mg per tablet.

In accordance with the invention, any suitable anti-tack agent can be employed in the second coating layer. An anti-tack agent is a compound that is used to reduce or minimize tackiness (i.e., stickiness) between coated tablets both during and after the tabletting process. Talc, glyceryl monostearate, and silica (colloidal) (or silicone dioxide) or mixtures thereof are examples of suitable anti-tack agents. Any suitable amount of the anti-tack agent can be used in the second coating layer, for example, about 10% or more, about 30% or more, or about 50% or more, or in an amount about 80% or less, about 60% or less, or about 40% or less, by weight of the methacrylic acid/methyl methacrylate copolymer. In certain embodiments, the anti-tack agent is present in an amount about 10% to about 85%, preferably about 30% to about 70%, and more preferably about 40% to about 60% by weight of the methacrylic acid/methyl methacrylate copolymer. The embodiment where the anti-tack agent represents about 40% to about 60% is preferred in the context of the present invention. In an embodiment, the anti-tack agent is present in an amount of about 50% by weight of the methacrylic acid/methyl methacrylate copolymer. Thus, for example, the anti-tack agent can be present in an amount about 2 mg or more, about 6 mg or more, or about 10 mg or more; or in amount about 12 mg or less, about 8 mg or less, or about 5 mg or less, and in embodiments, in an amount about 4 mg to about 16 mg, preferably about 6 mg to about 12 mg, and more preferably about 7 mg to about 11 mg per tablet.

The second coating layer can contain any suitable amount of plasticizer, for example, about 10% or more, about 20% or more, or about 30% or more, or in an amount about 25% or less, about 20% or less, or about 15% or less, by weight of the methacrylic acid/methyl methacrylate copolymer. In certain embodiments, the plasticizer is present in an amount about 12% to about 24%, preferably about 15% to about 22%, and more preferably about 17% to about 20% by weight of the methacrylic acid/methyl methacrylate copolymer. In an embodiment, the plasticizer is present in an amount of about 20% by weight of the methacrylic acid/methyl methacrylate copolymer. Thus, for example, the plasticizer can be present in an amount about 1 mg or more, about 2 mg or more, or about 3 mg or more; or in amount about 4 mg or less, about 3 mg or less, or about 2 mg or less; and in embodiments, in an amount about 1 mg to about 10 mg, preferably about 2 mg to about 8 mg, and more preferably about 3 mg to about 5 mg per tablet.

The second coating layer can include any suitable coloring agent, e.g., a pigment such as iron oxide yellow and/or iron oxide red, a lake or dye. The second coating layer can contain any suitable amount of coloring agent or agents, for example, about 10% or more, about 20% or more, or about 30% or more, or in an amount about 25% or less, about 20% or less, or about 15% or less, by weight of the methacrylic acid/methyl methacrylate copolymer. In certain embodiments, the coloring agent or agents are present in an amount about 10% to about 20%, preferably about 12% to about 18%, and more preferably about 14% to about 16% by weight of the methacrylic acid/methyl methacrylate copolymer. In an embodiment, the coloring agent or agents are present in an amount of about 15% by weight of the methacrylic acid/methyl methacrylate copolymer. Thus, for example, the coloring agent or agents can be present in an amount about 0.1 mg or more, about 2 mg or more, or about 3 mg or more; or in amount about 4 mg or less, about 3 mg or less, or about 2 mg or less, and in embodiments, in an amount about 0.2 mg to about 8 mg, preferably about 1 mg to about 6 mg, and more preferably about 2 mg to about 4 mg per tablet.

The second coating layer can be of any suitable loading, e.g., a loading of about 5 mg/cm² or more, and in embodiments, the second coating layer can have a loading of about 6 mg/cm² to about 15 mg/cm², preferably about 8 mg/cm² to about 14 mg/cm², and more preferably about 10 mg/cm² to about 13 mg/cm².

Optionally, a polishing layer may be added on top of the second coating layer. Suitably, the composition of the polishing layer comprises polyethylene glycol.

In accordance with an embodiment of the invention, the tablet releases at least about 50%, preferably about 60%, or more preferably about 70% by weight, of mesalazine from the tablet at pH 6.8 within 450 minutes when measured using USP dissolution apparatus II (paddle method), 100 rpm, pH 6.8 phosphate buffer according to USP reference standard USP30-NF25 (2007) or "the USP paddle dissolution test method".

In an embodiment, the invention provides a mesalazine delayed release tablet comprising a tablet core comprising mesalazine and an intergranular superdisintegrant, a first coating layer and a second coating layer disposed on the tablet core, the first coating layer comprising, consisting essentially of (e.g. comprises more than 90% by weight especially more than 95% by weight), or consisting of, hydroxypropyl methylcellulose and/or povidone, and the second coating layer comprising, consisting essentially of (e.g. comprises more than 90% by weight especially more than 95% by weight), or consisting of, poly(methacrylic acid, methyl methacrylate) 1:2 and about 40% to about 60% by weight of talc based on the amount of the poly(methacrylic acid, methyl methacrylate) 1:2.

The invention further provides a method for preparing a mesalazine delayed release tablet comprising:
(i) granulating a granulate composition comprising mesalazine, a pharmaceutically acceptable salt, or ester thereof, and a binder to obtain mesalazine granulates;
(ii) tabletting a core composition comprising the mesalazine granulates obtained in (i) and an intergranular superdisintegrant to obtain a tablet core;
(iii) coating the tablet core obtained in (ii) with a first coating layer by employing a coating composition comprising, consisting essentially of (e.g. comprises more than 90% by weight especially more than 95% by weight) or consisting of, a cellulose derivative and/or povidone to obtain a product; and
(iv) coating the product obtained in (iii) with a second coating layer by employing a coating composition comprising a methacrylic acid/methyl methacrylate copolymer and an anti-tack agent, e.g., wherein the amount of anti-tack agent is about 40% to about 60% by weight of the methacrylic acid/methyl methacrylate copolymer.

In particular, mesalazine, salt or ester thereof, is granulated before being compressed into a tablet. Suitably, the mesalazine granulate comprises the active ingredient in an amount of about 50% to about 95% based upon the total weight of the granulate.

The granulation step includes granulating the mesalazine or a pharmaceutically acceptable salt or ester thereof, and one or more pharmaceutically acceptable excipients by dry-granulation or wet granulation techniques. For example, granulation can be performed by wet granulation of a mixture of mesalazine, microcrystalline cellulose, and polyvinylpyrrolidone in water in a granulation apparatus. The granules obtained are dried and sieved to select the appropriately sized granules.

The tabletting step includes compression of the granules described above together with intergranular components to form a tablet core. Suitable shapes of the tablet core include round, oval shaped, conical, capsule shaped, and biconical. The coating steps include a first coating step of the core followed by a second coating step with the methacrylic acid/methyl methacrylate copolymer composition, and optionally a third coating step with a polishing composition. The coating layers can be applied utilizing any suitable method. For example, the coating layers are applied in an automated fluidized bed or a coating pan by spraying the coating composition in a solvent onto the tablet core. In this process, the water or organic solvent is removed by techniques known to one of ordinary skill in the art, e.g. by drying or during curing. The amount of enteric coating that is applied is sufficient to provide resistance to the acid environment in the stomach. By varying the amount of coating on the core, different dissolution profiles of the mesalazine tablets are obtained. In a preferred aspect of the present invention, at least about 50% of mesalazine, preferably about 60%, or more preferably about 70% by weight, is released from the tablet at pH 6.8 within 450 minutes.

The second coating layer comprises a methacrylic acid/methyl methacrylate copolymer and is applied in the form of a solution or suspension (collectively "dispersion") in a suitable solvent or a mixture of solvents. In a preferred embodiment, the coating dispersion has a solid content, for example, an average solid content, ranging from about 5% to about 20%, preferably from about 6% to about 15%, more preferably from about 7% to about 9% by weight of the dispersion. Suitable solvents include water, ethanol, methanol, isopropyl alcohol, chloroform, acetone, ether, or mixtures thereof. Preferably, the solvent is a mixture of ethanol and water. After the second coating, the coated tablets are dried under conventional conditions effective for drying, e.g., in an oven or by means of gas in a fluidized bed. As coated above, a strong continuous enteric coating layer is obtained that is smooth and that completely and uniformly or substantially uniformly surrounds the core.

The above method can be employed to produce a batch of tablets, wherein the batch of tablets show an intra-batch variation dt₅₀ of less than about 200 minutes, preferably less than about 160 minutes, and more preferably less than about 120 minutes, e.g., from about 100 to about 120 minutes, wherein dt₅₀ is the difference in the time required to release 50% of mesalazine from the quickest tablet to the slowest tablet when 6 tablets of the same batch are tested for dissolution in a phosphate buffer at pH 7.0 and 37 °C according to the USP paddle dissolution test method.

Accordingly, the present invention provides a method for preparing a batch of mesalazine delayed release tablets having uniformity in terms of release characteristics, the method comprising:
(i) granulating a granulate composition comprising mesalazine and a binder to obtain mesalazine granulates;
(ii) tabletting a tablet core composition comprising the mesalazine granulates obtained in (i) and an intergranular superdisintegrant to obtain tablet cores;
(iii) coating each of the tablet core with a first coating layer by employing a coating composition comprising a cellulose derivative and/or povidone to obtain coated tablet products; and
(iv) coating each of the coated tablet products obtained in (iii) with a second coating layer by employing a coating composition comprising a methacrylic acid/methyl methacrylate copolymer and an anti-tack agent, e.g., wherein the amount of anti-tack agent is about 40% to about 60% by weight of the methacrylic acid/methyl methacrylate copolymer, to obtain the batch of delayed release tablets;
wherein the batch of tablets shows an intra-batch variation dt₅₀ less than about 200 minutes, wherein dt₅₀ is the difference in the time required to release 50% of mesalazine from the quickest tablet to the slowest tablet when 6 tablets of the batch are tested for dissolution in a phosphate buffer at pH 7.0 and 37 °C according to the USP paddle dissolution test method.

Batches of tablets produced in accordance with the invention are highly uniform, wherein the inter-batch variation is minimal. Thus, for example, the invention provides a method of preparing multiple batches of tablets which includes repeating (i)-(iv) to produce multiple batches of mesalazine delayed release tablets, wherein the multiple batches show an inter-batch variation DT₅₀ less than about 150 minutes, e.g. less than about 140 minutes, preferably less than about 120 minutes, more preferably less than about 75 minutes, such as 50, 40, 30, or 20 minutes or less, wherein DT₅₀ is the difference in the average time to release 50% of the mesalazine (at₅₀) between the slowest batch and the quickest batch when tablets from 3 batches are tested for dissolution in a phosphate buffer at pH 7.0 and 37°C according to the USP paddle dissolution test method, wherein the average time of release of batch "j" is at^{j}₅₀ = Σ (tⁱ₅₀)/6, wherein i=1 to 6, and tⁱ₅₀ is the time to release 50% of the drug from tablet "i" of batch "j" when 6 tablets of the batch "j" are tested for dissolution in a phosphate buffer at pH 7.0 and 37°C according to the USP paddle dissolution test method. Thus, in accordance with the invention, DT₅₀ = at₅₀ slowest - at_{50 quickest}.

The invention also provides tablets and batches of tablets produced in accordance with embodiments of the invention. The present invention further provides a method of treating a patient for an inflammatory bowel disease (IBD) comprising administering to the patient an effective amount of a mesalazine delayed release tablet described above.

The invention also provides a batch of delayed release tablets comprising a plurality of delayed release mesalazine tablets, wherein the batch shows an intra-batch variation dt₅₀ less than about 200 minutes, preferably less than about 160 minutes, and more preferably less than about 120 minutes, e.g., from about 100 to about 120 minutes,
wherein dt₅₀ is the difference in the time required to release 50% of mesalazine from the quickest tablet to the slowest tablet when 6 tablets of said batch are tested for dissolution in a phosphate buffer at pH 7.0 and 37°C according to the USP paddle dissolution test method. The invention further provides a set comprising multiple batches of delayed release tablets of mesalazine, each of said multiple batches of delayed release tablets comprising a plurality of mesalazine tablets, said set showing an inter-batch variation DT₅₀ less than about 150 minutes, e.g. less than about 140 minutes, preferably less than about 120 minutes, and more preferably less than about 75 minutes, such as 0, 40, 30, or 20 minutes or less, wherein DT₅₀ is the difference in the average time to release 50% of the mesalazine (at₅₀) between the slowest batch and the quickest batch when 3 batches are tested for dissolution in a phosphate buffer at pH 7.0 and 37°C according to the USP paddle dissolution test method, wherein at^{j}₅₀ = Σ (tⁱ₅₀)/6, wherein i=1 to 6 and tⁱ₅₀ is the time to release 50% of the drug from tablet "i" of batch "j" when 6 tablets of batch "j" are tested for dissolution in a phosphate buffer at pH 7.0 and 37°C according to the USP paddle dissolution test method. DT₅₀ = at_{50 slowest} ∼ at_{50 quickest}.

The following example further illustrates the invention but, of course, should not be construed as in any way limiting its scope.

### EXAMPLE

This Example illustrates a method of preparation of delayed release tablets according to an embodiment of the invention.

A wet granulate of mesalazine, microcrystalline cellulose, colloidal silicon dioxide and polyvinylpyrrolidone is prepared. After drying, the granules are sieved through a sieve. Subsequently, the sieved granules are blended with microcrystalline cellulose, cross-linked polyvinylpyrrolidone, and magnesium stearate or sodium stearyl fumarate. The resulting blend is compressed to obtain tablet cores.

The tablet cores (1 kg) are pre-coated by spraying a HPMC/PEG solution with a solid content of 4% by weight. Subsequently, the second coating layer is applied by spraying a dispersion of EUDRAGIT® S, talc, triethylcitrate and the coloring agents in ethanol/water onto the pre-coated tablets. The solid content in the dispersion is 8% by weight. The coated tablets are dried in a rotating pan.

Two sets of batches of tablets are prepared under conditions as indicated above, resulting in the compositions set forth in Table 1. The intra-batch variation (dt₅₀) values and the inter-batch variations (DT₅₀) values of batches of the tablets are shown in Table 2.

**Table 1. Composition of batches of mesalazine tablets**

| Composition | Batch 218339/ 218340/ 218341 | | Batch 218356/218357/ 218358 | |
|---|---|---|---|---|
| | (mg) | (%) | (mg) | (%) |
| ***Tablet core*** | | | | |
| Mesalazine | 400 | 76.2 | 400 | 76.2 |
| Microcrystalline cellulose | 65.4 | 12.5 | 59.7 | 11.4 |
| Amorphous SiO₂ in water | 4.8 | 0.9 | 4.8 | 0.9 |
| Povidone | 28.8 | 5.5 | 28.8 | 5.5 |
| Crospovidone | 21 | 4.0 | 26.2 | 5.0 |
| Mg stearate | 4.8 | 0.9 | - | - |
| Sodium stearyl fumarate | - | - | 5.3 | 1.0 |

| ***First Coating Layer*** | | | | |
|---|---|---|---|---|
| HPMC | 3.5 | - | 3.5 | - |
| PEG | 0.5 | - | 0.5 | - |

| ***Second Coating Layer*** | | | | |
|---|---|---|---|---|
| Eudragit S 100 | 20.3 | - | 20.3 | - |
| Talc | 10.1 | - | 10.1 | - |
| Triethylcitrate | 4 | - | 4 | - |
| Iron oxide yellow | 0.5 | - | 0.5 | - |
| Iron oxide red | 2.7 | - | 2.7 | - |

**Table 2. The dt₅₀ and DT₅₀ values of tablets shown in Table 1.**

| pH 7.0 | | | |
|---|---|---|---|
| Batch | dt₅₀ (min.) | Batch | dt₅₀ (min.) |
| 218339 | 119 (228-347) | 218356 | 66 (135-201) |
| 218340 | 160 (158-318) | 218357 | 124 (137-261) |
| 218341 | 106(199-305) | 218358 | 103(130-233) |
| | DT₅₀ (min.) | | DT₅₀ (min.) |
| | 55(272-217) | | 20(192-172) |

Subsequently, to some coated tablet samples a polishing coat is applied by spraying a PEG solution. All tablets are post-dried for example in a drying cabinet. All tablets show a smooth coating layer. The dissolution profiles of the individual tablets are determined using USP dissolution apparatus II (paddle method), 100 rpm, pH 7.0 phosphate buffer (according to USP reference standards USP30-NF25). The dissolution profile of the tablets of Batch No. 218357 is measured at pH 6.8 to show that the tablets of the invention release the drug even at a pH of 6.8 and the dissolution profile is shown in the Figure. I did not get the figure the first time I reviewed the application, but I can see now that the time difference at pH-.8 is from 200min to 450min, i.e. this is a quite large number. Why is the number so high at pH 6.8 compared to what is claimed at pH7.0?

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## Claims

1. A mesalazine delayed release tablet comprising a tablet core, a first coating layer being in contact with and covering the tablet core, and a second coating layer being in contact with and covering the first coating layer, wherein
the tablet core comprises mesalazine, a binder, and at least one intergranular superdisintegrant;
the first coating layer which is free or substantially free of methacrylic acid/methyl methacrylate copolymer and which comprises a cellulose derivative and/or povidone; and
the second coating layer comprises a methacrylic acid/methyl methacrylate copolymer and an anti-tack agent, wherein the amount of anti-tack agent is present in an amount about 40% to about 60% by weight of the methacrylic acid/methyl methacrylate copolymer.

2. The tablet according to claim 1, wherein the intergranular superdisintegrant is selected from the group consisting of crospovidone, croscarmellose sodium, sodium starch glycollate, and mixtures thereof, preferably crospovidone.

3. The tablet according to claim 1 or 2, wherein the binder is selected from the group consisting of microcrystalline cellulose, polyvinylpyrrolidone, lactose, starch, and hydroxypropyl cellulose, and mixtures thereof.

4. The tablet according to any one of claims 1 to 3, wherein the anti-tack agent is selected from the group consisting of talc, glyceryl monostearate, silica and mixtures thereof.

5. The tablet according to any one of claims 1 to 4, wherein the cellulose derivative of the first coating layer is hydroxypropyl methylcellulose (HPMC).

6. The tablet according to any one of claims I to 5, wherein the methacrylic acid/methyl methacrylate copolymer is poly(methacrylic acid, methyl methacrylate) 1:2.

7. The tablet according to any one of claims 1 to 6, wherein the first coating layer is present in an amount of about 0.5% to about 1.5% by weight of the tablet and the second coating layer is present in an amount of about 3% to about 10% by weight of the tablet.

8. The tablet according to any one of claims 1 to 7, wherein the tablet core further includes one or more excipients selected from the group consisting of glidants, lubricants, fillers, and wetting agents.

9. The tablet according to any one of claims 1 to 8, wherein the first coating layer further includes a polyol, preferably polyethylene glycol.

10. The tablet according to any one of claims 1 to 9, wherein the second coating layer further includes one or more additives selected from the group consisting of plasticizers and coloring agents.

11. The tablet according to any one of claims 1 to 10, which releases at least about 50% of mesalazine from the tablet at pH 6.8 within 450 minutes when measured using USP dissolution apparatus II (paddle method), 100 rpm, pH 6.8 phosphate buffer according to USP reference standard USP30-NF25 (2007).

12. A batch of delayed release tablets comprising a plurality of delayed release mesalazine tablets, wherein the batch shows an intra-batch variation dt₅₀ less than about 200 minutes, wherein dt₅₀ is the difference in the time required to release 50% of mesalazine from the quickest tablet to the slowest tablet when 6 tablets of said batch are tested for dissolution in a phosphate buffer at pH 7.0 and 37°C according to the USP paddle dissolution test method.

13. The tablet of claim 12, wherein the dt₅₀ is less than about 160 minutes, preferably less than about 120 minutes.

14. A set comprising multiple batches of delayed release tablets of mesalazine, each of said multiple batches of delayed release tablets comprising a plurality of mesalazine tablets, said set showing an inter-batch variation DT₅₀ less than about 75 minutes wherein DT₅₀ is the difference in the average time to release 50% of the mesalazine (at₅₀) between the slowest batch and the quickest batch when 3 batches are tested for dissolution in a phosphate buffer at pH 7.0 and 37°C according to the USP paddle dissolution test method, wherein the average time of release of tablets from batch "j": at^{j}₅₀ = Σ (tⁱ₅₀)/6, and i=1 to 6, wherein tⁱ₅₀ is the time to release 50% of the mesalazine from tablet "i" of batch "j" when 6 tablets of the batch "j" are tested for dissolution in a phosphate buffer at pH 7.0 and 37°C according to the USP paddle dissolution test method.

15. The tablet, batch of tablets or set of multiple batches of any one of claims 1 to 14, for the treatment of inflammatory bowel disease (IBD).

16. A method for preparing a mesalazine delayed release tablet comprising:
(i) granulating a granulate composition comprising mesalazine and a binder to obtain mesalazine granulates;
(ii) tabletting a core composition comprising the mesalazine granulates obtained in (i) and an intergranular superdisintegrant to obtain a tablet core;
(iii) coating the tablet core obtained in (ii) with a first coating layer composition comprising a cellulose derivative and/or povidone to obtain a product; and
(iv) coating the product obtained in (iii) with a second coating layer by employing a coating composition comprising a methacrylic acid/methyl methacrylate copolymer and an anti-tack agent and wherein the amount of anti-tack agent is about 40% to about 60% by weight of the methacrylic acid/methyl methacrylate copolymer, and
optionally repeating steps (i)-(iv).

17. The method according to claim 16, wherein the coating composition employed to form the second coating layer is a suspension having a solids content of about 6% to about 12% by weight of the suspension.

18. The method according to claim 16 or 17, for the preparation of the tablet, batch of tablets or set of multiple batches of any one of claims 1 to 15.
